# EUROPEAN PATENT APPLICATION

(11) **EP 0 538 259 A1**
(43) Date of publication of application: **28.04.1993**
(21) Application number: 91904833.0
(22) Date of filing: 28.02.1991
(51) Int. Cl.: A61B 17/34, A61B 5/03

(54) **METHOD AND APPARATUS FOR LOCATING ANATOMICAL CAVITIES**

(71) Applicant: INDUSTRIAS PALEX, S.A., E-08021 Barcelona (ES)
(72) Inventor: UCHA CALVO, Eloy, E-08181 Rubi (ES)
(74) Representative: Ponti Sales, Adelaida
(86) International application number: ES9100011
(87) International publication number: WO9215256

(57) **Abstract**

The present invention relates to a method and apparatus for locating anatomical cavities such as the epidural space. The puncture will be made on the basis of the loss resistance technique with a syringe (12). The tapping is facilitated by taking some measurements in the operation region (15) and by the emission of acoustic and visual warning signals, as well as by the provision of data displayed on a screen (16), all of which quantify and corroborate the tactile feelings of the operator, influencing the operator's work and allowing the operator to make proximal trials which are not harmful or damaging, and to be extremely sure that the cavity has been reached. The method is applicable independently of the pressure characteristics of the space to be detected, and uses a conventional needle, which also makes an apprenticeship possible in optimal safety conditions.

## Description

This invention relates to a method for locating anatomical cavities, such as the epidural space, pleural, peritoneal cavities, and other similar ones, through a needle that is inserted through the tissues surrounding such area until its tip reaches the specific space, and an apparatus to implement such method.

### SCOPE OF THE INVENTION

We know very well the importance in the modern medical practice of locating anatomical cavities such as the epidural space and other similar ones, either for introducing in such intra-corporal space anaesthetics, analsegics or drugs, by injecting them through a needle or a catheter, or for extracting solutions residing in such cavities for exploration and/or analysis for the diagnosis of different diseases. In particular, and with reference to the epidural space, we can see that the epidural anaesthetic, due to its many advantages and, especially, due to the fact that it allows long anaesthetic periods, is currently one the most commonly used loco-regional anaesthetic techniques, not only for surgical and obstetrics purposes, but for diagnosis, prognosis and treatment of different types of acute and/or chronic pain.

Furthermore, we know the problems resulting from implementing the different techniques which are currently in use, with the support or different devices and/or apparatuses applied for that purpose.

The difficulties of a sure implementation to locate, precisely and without damage, an anatomical cavity result mainly from the fact that the depth of penetration in order to reach such cavity and the size of the same differs from one patient to another and is influenced by many factors such as age, degree or level of development, obesity, sex, and different thickness of the tissues between the skin and the interior of the cavity. Furthermore, the characteristics of the cavities (size, internal pressure, shape, among others) differ in the various regions, for example, in the case of an epidural cavity, the cervical and high dorsal regions are different from the lumbar and sacrum.

An excessive penetration in the cavity, which is easy to occur, due to a sudden drop in the resistance once the wall of the cavity has been penetrated, may result in lesions to the internal organs which, in the case of the epidural cavity translate mainly into punctures to the dura mater or the fibres of the nervous fascicle.

### BACKGROUND OF THE INVENTION

The main methods of identification that are known can be divided into two groups:
a. methods based on a specific existing pressure in the cavity to be located (for example, a negative pressure or depression in the epidural space), and
b. methods based on the loss of resistance.

The most common methods and the apparatus used to implement them, together with the problems that result from them, are described in different previous patents and technical literature which are detailed herein below.

Thus, based on the first method and as their more significant examples, we may mention American U.S. patents 4.175.567 and 4.215.699, where the depression located in the epidural space is used to flex a membrane which is in contact with the needle duct and thus gives a visual indication to the operator. The use of this technique is limited to the presence or a sufficient negative pressure in the cavity to locate. There is the possibility of an obstruction of the needle resulting in difficulty of communication between the epidural space and the membrane. The sensitivity of the anaesthetist is not used in the operation.

The European patent No. 8303524 which detects the aforementioned depression through a sensor in contact with the needle duct, associated with a circuit that activates an electromagnet, through which such needle is brought back into a coaxial cannula so that the latter covers its tip. The use of the apparatus is limited to a negative pressure in the cavity to be detected. This leads to failures in the case of areas crossed during the advance, with expansions that create decreases in pressure, and due to obstructions to the needle that make the communication between the space to detect and the sensor difficult. Furthermore, the unit is complex, with a non-conventional needle. It does not take advantage of the sensitivity of the operator, and the relative shifting of the needle may cause errors and false positioning.

Another technique is the one based on the visualization of a drop of anaesthetic placed in the back pavilion of the needle, which shall be absorbed when the tip of the needle reaches the epidural space. A similar method is described in U.S. patent 4.162.673, where a small quantity of liquid shifts, due to the aforementioned depression, from a cavity towards a duct. Furthermore, this technique has been used with the drop of liquid in a crystal capillary tube in order to be able to better visualize the absorption of such drop. This technique is not useful when there is a vascular puncture or a partial obstruction of the tip of the needle while going through the tissues. When the advancement of the needle is intermittent, it results in a high level of dural punctures.

With regard to the techniques based on the loss of resistence, we may mention U.S. patent 4.535.773, where a case which surrounds the needle coaxially, except for its front end, covers it completely, pushed by a spring, when it reaches the cavity and is allowed to shift. The apparatus is extremely complex and is not useful due to major limitations in the cervical and high dorsal areas.

The technique which is known as loss of resistance, which consists of attaching a syringe with some cubic centimetres of physiological serum to the needle at the time of puncture, applying a slight continuous pressure on its plunger, detecting the epidural space when the resistance offered by the plunger to the injection decreases. The technique may lead to error if the end of the needle finds very slack tissues offering less resistance to the injection; and requires training and experience on the part of the anaesthetic. In spite of all these problems, this is one of the most common methods used.

The MacIntosh balloon method consists of attaching a soft rubber balloon to the pavilion of the needle, and proceeding with blowing it with several cubic centimetres of air. At the time when the tip of the needle is introduced in the epidural space, and due to the existence or a vacuum in it, the rubber balloon shall abruptly deflate. The technique may be used at all levels of the spine, but leads to errors if the tip of the needle finds a blood vessel (danger of air embolism), if the tip of the needle is in the para-vertebral space due to its slackness or if the patient is very slim.

As an alternative to these techniques and apparatus, U.S. patents 4.623.335 i 4.801.293 describes a method where an inflatable balloon and a mechanical device with a spring for pressure detection are used, installed in a derivation of an associated valvular body between the tip of the needle and a syringe to inject anaesthetic. As the needle advances through the tissues, the operator must inflate and deflate the said balloon with the purpose of maintaining a substantially constant pressure in the closed system, air-air and, when the tip of the needle reaches the epidural space, there is a drop of pressure that is detected by the sudden shift of a plunger supported by the spring.

This technique requires very complex and/or delicate handling on the part of the operator who must insert the needle at the same time he/she pumps air and observe the pressure scale. This may lead to errors which are similar to the ones described in the McIntosh balloon technique, and does not take advantage of the sensitivity of the operator. Besides, since it is based on the introduction of a certain amount of air inside the cavity, this may result in negative effects for the patient in the case of the epidural space (strong headaches).

### SUMMARY OF THE INVENTION

The purpose of the invention is a method to locate anatomical cavities taking advantage of the aforementioned techniques, and eliminating inconveniences caused by them, requiring a minimum of attention on the part of the operator, who shall be able to concentrate in implementing the puncture which is carried out based on the aforementioned technique of loss resistance with a syringe, assisted by measurements taken in the working area and the emission of acoustic and visual warning signs, being different depending on whether the needle has reached an area close to an epidural space or is actually located in the epidural space, as well as by the display of data on a screen, all of which quantify and corroborate the tactile feelings of the operator, influencing the operator's work and allowing him/her to perform proximal or feeling tests which are not detrimental, thus obtaining a good assurance that the cavity has been reached.

The method may be applied independently from the pressure characteristics of the space to be detected, and it uses conventional needle, further allowing training under the safest conditions since it allows the operator to perform proximal test which are not harmful.

An apparatus has been created for this purpose, which consists of a needle of length and width which are adequate to penetrate through the tissues of the area to explore and reach the cavity to be located with its tip; a syringe; a "T" connector, and conventional means of attachment with a switch which can establish communications among all its ducts; means for perception and measurement of the pressure of the internal liquid of the syringe and display on a screen; means for processing the information supplied by the pressure measurement means which allow the measurement of the speed of the pressure variations originated as the needle/syringe unit advances; and signalling means which include, at least, an electroacoustic device and a pilot-light.

The proposed method includes a series of stages which are common to a technique of loss resistance with monitored pressure 1. sticking and inserting the aforementioned needle towards the cavity until it is positioned in an area which is close to the tissues surrounding the cavity; 2. preparing the "T" connector with a switch capable of communicating its ducts with one another, joining the first of such ducts to the female connector of the back tip of the needle, and a second duct of the "T" connector to a tube connector that links with means that detect and indicate pressure variations; 3. attaching the syringe loaded with a limited, reduced amount of an isotonic solution, such as a physiological serum, to the free opening or the "T" connector. The method consists of the following phases/stages: 4. communicating the inside of the syringe, the axial connector of the needle and the aforementioned tube connector with the means that detect and indicate pressure, and calibrating the reading pressure displayed on the screen to zero, when there is not pressure on the plunger of the syringe; 5. increase the pressure of the liquid, which is internal both to the syringe and the needle, in a practical closed system, pressing the plunger of the syringe until the pressure shown on the display screen exceeds a predetermined threshold; 6. pressing the unit slowly raising the needle through the tissues close to the anatomical cavity and, at the same time, maintaining constant pressure on the plunger that shall be corrected with the assistance of the pressure reading of the liquid column on the display screen; 7. performing, by means of the appropriate device associated with the aforementioned pressure-measuring means, a data collection concerning the pressure of the liquid in the syringe, and detecting pressure variations by measuring the speed of the pressure variations occurring during the slow advance of the needle towards the anatomical cavity, through the means provided for that purpose; 8. transmitting, through means associated with the aforementioned measuring end detection ones, a first warning signal which is advantageously electroacoustic, intermittent, with pre-determined frequency and amplitude, in response to a specific drop in pressure at the aforementioned liquid column, and to a speed in such pressure variation which falls within preset margins. This signal shall stop when the pressure of the system is capable of recovery by means of a slight pressure placed on the plunger, without advancement of the needle, continuing with a slow advance; 9. transmitting, through means associated with the aforementioned measuring and detection ones, a second warning signal which is advantageously electroacoustic, intermittent, clearly different from the previous one in frequency and amplitude, in response to a more abrupt drop in pressure that cannot be recovered through a slight pressure on the plunger, without advancement of the needle, at which time the insertion or such needle shall stop; 10. separating the "T" connector-syringe unit from the needle with its tip already positioned in the anatomical cavity in question, and attaching to the needle connector a syringe or any other device for the required operation.

In the apparatus, the measuring devices of the internal pressure of the liquid include a piezoelectric type pressure sensor or group of sensors.

The means for the processing of the information supplied by the aforementioned pressure measurement means include an amplification stage of the pressure signal, attached to a digital analogical converter connected to the input of a microprocessor dedicated to the automatic control of the process that governs the outputs of the display screen, an electroacoustic warning device and a pilot-light, with a control program storage memory and a second memory to store calibrating data and detection points. The system includes a device to reset the unit and start the process.

### DESCRIPTION OF THE DRAWINGS

The attached drawings, which serve only as an example, shall give a better understanding of the invention, its characteristics and the advantages that it may bring:
FIGURE 1 is a horizontal section view of the apparatus with the needle inserted in the human body in the position of locating a cavity, provided with a "T" connector, a syringe and means for perception and pressure measurement.
FIGURE 2 is a view which is similar to the previous one in the position of having located a cavity.
FIGURE 3 is a block diagram of the means of information processing supplied by the pressure measuring means.

The items designated with numbers on the drawings correspond to the following parts.

### PREFERRED PERFORMANCE OF THE INVENTION

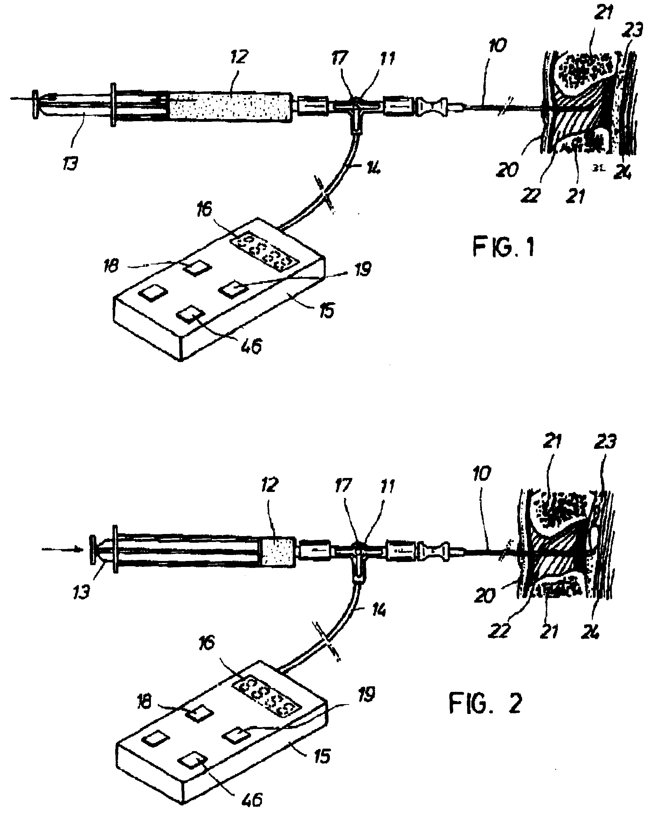

As an example of the preferred performance of the invention, we would like to refer to a practical example of locating an epidural cavity. Figures 1 and 2 represent a section of the patient's body through the lumbar region.

The epidural cavity (23) is physically located between the skin of the patient (20), the tissues (22), the vertebrae (21) and the dura mater (24).

Both figures represent the needle (10) introduced through its tip into the patient's body through the skin (20), and the tissues (22) between the vertebrae (21), linked on its other end to the "T" connector (11), which shows attached to its other two branches the syringe (12) with its plunger (13), and the connector (14) associated with the means (15) for perception and pressure measurement of the liquid column inside the syringe as well as for indicating such on the display screen (16).

The "T" connector (11) includes the aforementioned three projections connected to the noodle (10), the syringe (12) and the connector (14), and there is a plug (17) that allows for the closing and passage control among the three aforementioned branches.

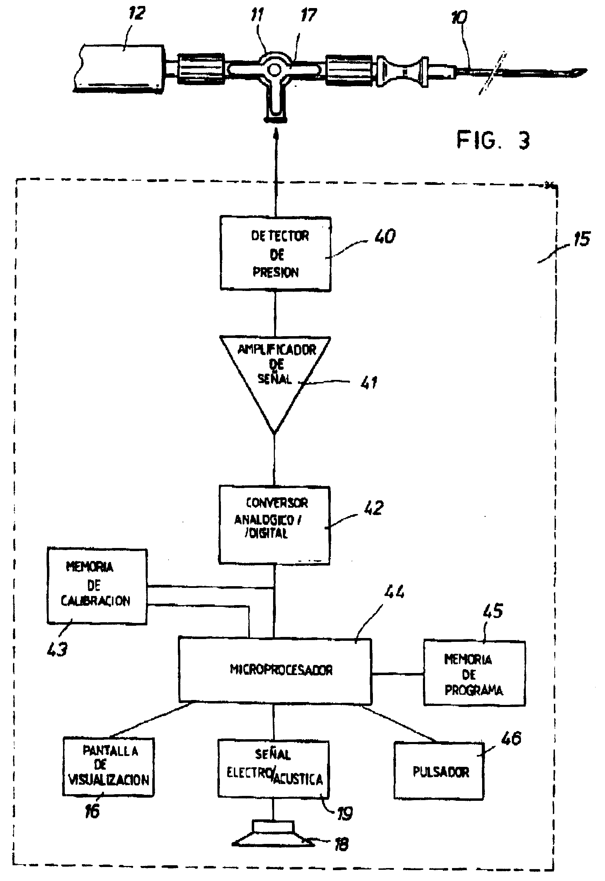

The means (15) for the processing of the information provided by the pressure measuring ones include, as appears on figure 3, a pressure detector (40), an amplifier (41) of the pressure sensor signal, an analogical/digital converter (42), a microprocessor (44), program memory (45), calibrating memory (43), a reset switch (46) and means for displaying (16), electro/acoustic signals (18) and light signals (19).

The method which includes this invention consists of sticking and inserting the needle (10) towards the epidural cavity (23) to be located, until it is positioned close to the tissues (22) surrounding such cavity, with a "T" connector (11) with access through its ducts and conventional means of attachment at the openings, joining one of the ducts to the connector at the back tip of the needle (10), a second projection to a tube connector (14), and a third linked to the tube connector (14), with the plug (17) in the closed position, attaching the syringe (12) loaded with a limited and reduced amount of physiological serum, to the free projection or the "T" connector (11).

In this position, it is necessary to establish a communication, by operating the aforementioned plug (17), among the inside of the syringe (12), the needle (10), and the aforementioned tube connector (14) that links the means to detect and indicate pressure, and to calibrate the reading pressure shown on the display screen (16) to zero with the switch (46).

Then the pressure of the practically closed system shall be increased by pressing on the plunger (13) of the syringe (12). The pressure that is obtained is shown on the display screen (16). By pressing the unit, and slowly advancing the needle (10) through the tissues (22) close to the epidural cavity (23), a constant pressure on the plunger (13) shall also be maintained, and this shall be confirmed on the display screen (16). Then, with the reading device associated with the aforementioned pressure measuring means (40), we shall be able to obtain, at regular intervals, data concerning the pressure of the liquid in the syringe (12), and, in particular, detect pressure variations by measuring the speed of the pressure drops that shall occurs during the slow advancement of the needle (10) towards the epidural cavity (23), with the conventional means provided for that purpose.

In response to a specific drop in pressure in the aforementioned liquid column and/or a pressure variation that falls between minimum margins, the means (15) for perception and pressure measurement shall detect it, transmitting a first intermittent warning signal through the electroacoustic means (18).

This signal shall stop if the pressure of the system can recover through a slight pressure on the plunger, without advancement of the needle.

On the other hand, if there is a drop in the pressure and it cannot recover through a slight pressure on the plunger, the electroacoustic means (18) shall continue with the warning signal, thus confirming that the epidural cavity (23) has been located.

In this situation, the "T" connector (11), the syringe (12) and the devices (15) for perception and pressure measurement may be withdrawn. With this, the tip of the needle (10) shall remain free for attaching a second syringe or inserting a catheter or any other means for the operation required.

Figure 3 shows a diagram of the different blocks forming the associated means (15) for perception and pressure measurement. In particular, this section includes a piezoelectric type pressure sensor or group of sensors (40).

These are assisted by an amplification stage of the measuring signal (41) attached to the digital analogical converter (42) connected to the inputs of a microprocessor (44) dedicated to the automatic control of the process regulating the output at a display screen (16), an electroacoustic warning device (18) and a pilot-light (19). It also has storage memory for a control program (45) and a second storage memory for calibrating data and detection points (43). The system includes a switch to set the reading unit back to zero (46) and start the process.

The component of these associated means (15) for detection and pressure measurement are of the conventional type, laid out in an advantageous manner and fed by a low voltage energy source. They may be placed in only one surrounding casing, for convenience purposes.

## Claims

1. Method for locating anatomical cavities, such as the epidural space, pleural, peritoneal cavities, or similar cavities with a needle, for the purposes of injecting analgesic solutions or for the extraction of samples through it, and for dilating the said intracorporal space in the proximity of the tip of such a needle, prior to the injection of such solutions, which consists of attaching to a needle (10), after inserting it in the patient's body towards the cavity (24), until it is positioned in the area close to the tissues (22) surrounding such anatomical cavity (24), a syringe (12) loaded with a limited, reduced amount of isotonic solution, such as the physiological serum, on the free projection of the connector of such needle (10), applying a slight continuous pressure on its plunger (13), and detecting the anatomical cavity (24) when the resistance of the said plunger (13) to the injection is diminished. This syringe (12) is connected to pressure detecting devices (15) through a "T" connector (11) with a switch (17) that allows it to whether or not establish communication among the three ducts, characterized by the following stages.
a. to establish communication, by operating the aforementioned switch (17), among the inside of the syringe (12), the axial connector of the needle (10) and the third tube connector (14) of the "T" connector (11), linked to detecting and pressure indicating means (15), to calibrate the reading pressure appearing on the display screen (16) to zero when there is no pressure on the plunger (13) of the syringe (12), and to press such plunger (13) of the syringe (12) until the pressure shown on the display screen (16) surpasses a predetermined threshold;
b. to push the syringe-needle unit causing the needle (10) to advance slowly through the tissues (22) that are close to the anatomical cavity (24), maintaining the said pressure, which is shown on the display screen (16), substantially constant by acting on the plunger (13) of the syringe (12);
c. to perform data collection at regular intervals concerning the pressure of the liquid column in the syringe (12), with the purpose of detecting pressure variations and measuring the speed with which such pressure variations occur during the slow advancement of the needle (10) towards the anatomical cavity (24), to transmit, in the presence of a drop in the pressure of the said liquid, with a speed of such pressure variation between the preset margins, through means associated with those of detection and measurement ones (15) mentioned above, a warning signal (18), at which time the advancement of the syringe-needle unit shall stop and which signal shall adopt two different possibilities:
c1. a first warning signal (18) which is advantageously electroacoustic, intermittent, with pre-determined frequency and amplitude, which shall stop if the pressure can return to a constant recovery to the preset value, by means of a slight pressure placed on the plunger (13), without advancement of the needle (10), in which case the slow advancement of the syringe-needle unit shall then be continued; or
c2. a second warning electroacoustic, intermittent, signal, clearly different from the previous one in frequency and amplitude, in response to an abrupt drop in pressure that cannot be recovered through a slight pressure on the plunger (13), without advancement of the needle (10), at which time the insertion of such needle (10) shall stop since its tip will have reached the anatomical cavity (24).

2. Method, according to the aforementioned claim, characterized by the fact that during the detection phase of an abrupt drop in pressure, such occurrence is additionally indicated by the lighting of a pilot-light (19) which remains permanently on until the "T" connector and syringe unit is disconnected from the needle (10).

3. Apparatus for locating anatomical cavities which includes:
a needle (10) of adequate length and section to penetrate through the tissues (22) of the area to explore, and reach the cavity (24) to be located with its tip that has no sharp edges that could damage such tissues (22);
a syringe (12) loaded with a limited, reduced amount of an isotonic solution, such as physiological serum;
a "T" connector (11) with a switch (17) capable of establishing communication among its three ducts, with the two lined branches attached respectively to the front tip of the syringe (12) and the female connector of the back tip of the needle (10), and with its third duct linked to a tube connector (14);
means (15) for perception and pressure measurement of the internal liquid of the syringe (12) and for indicating such on a display screen (16), which are associated with that tube connector (14);
characterized by integrating:
a. means for the processing of the information supplied by the pressure measuring means (15), applied to a date collection, taken at regular intervals, of the pressure values of the internal liquid in the syringe (12) and the needle (10), and to the calculation of the speed of the pressure variations that may occur;
b. signalling means (18-19) including, at least, an electroacoustic device (18) and a pilot light (19) which may be activated by the aforementioned means (15) of information processing, according to, at least, two clearly different operational cycles.

4. Apparatus, according to claim 3, characterized by the fact that the means for processing the information supplied by the aforementioned means (15) of perception and pressure measurement include an amplification stage (41) of the signal from a pressure sensor attached to a digital analogical converter (42) connected to one of the inputs of a microprocessor (44) dedicated to an automatic control of the process, which regulates the outputs of a display screen (16), an electroacoustic warning device (18) and a pilot-light (19); storage memory (45) for a control program and a second storage memory (43) for calibrating data and detection points; and a device (46) to reset the reading unit and start the process.
